# EUROPEAN PATENT APPLICATION

(11) **EP 3 960 723 A1**
(43) Date of publication of application: **02.03.2022**
(21) Application number: 20796214.3
(22) Date of filing: 24.04.2020
(51) Int. Cl.: C05F 11/08

(54) **LIQUID BIOFERTILISER WHICH COMPRISES AZOSPIRILLUM BRASILENSE AND PANTOEA DISPERSA STRAINS AND METHOD FOR OBTAINING SAME**

(30) Priority: 26.04.2019 ES 201930369
(71) Applicant: PROBELTE S.A.U., 30100 Murcia (ES)
(72) Inventor: MALO LÓPEZ-ROMÁN, Jorge, 30100 Murcia (ES); MENGUAL SOTO-NAVARRO, Carmen María, 30100 Murcia (ES); GARCÍA CARPENA, Juan José, 30100 Murcia (ES); CARRILLO GÓMEZ, Arturo Jesús, 30100 Murcia (ES); BOTELLA CAYUELAS, Alberto, 30100 Murcia (ES); BLANCA PICÓ, Isidro, 30100 Murcia (ES); CASANOVA BELMONTE, José Manuel, 30100 Murcia (ES); VÁZQUEZ FERNÁNDEZ, Guillermo, 30100 Murcia (ES)
(74) Representative: Manuel Illescas y Asociados, S.L.
(86) International application number: PCT/ES2020/070259
(87) International publication number: WO 2020/216978

(57) **Abstract**

A liquid biofertiliser which comprises *Azospirillum brasilense* CECT 5802 and *Pantoea dispersa* CECT 5801 strains. A method for stimulating plant growth, which comprises applying said liquid biofertiliser to said plant. A method for obtaining a liquid biofertiliser, which comprises culturing said strains in first liquid culture mediums and adding the culture broths obtained to a second liquid culture medium.

## Description

### TECHNICAL FIELD OF THE INVENTION

The present invention relates to a product for biological fertilisation consisting of a liquid formulation containing two strains of bacteria of the genera *Azospirillum* and *Pantoea* capable of fixing atmospheric nitrogen, solubilising phosphates as well as other mineral nutrients from the soil, and producing high amounts of plant growth stimulating substances. Such microorganisms have been formulated in liquid form, ensuring easy application and high stability in cell viability, and providing sufficient organic and mineral nutrients to facilitate plant root colonization.

### BACKGROUND OF THE INVENTION

The use of fertilisers is essential for the maintenance of high yields in crops. By means of chemical fertilisation, significant amounts of nitrogen, phosphorus, and potassium as well as other mineral elements are added to the soil. However, their availability is very low, since a fraction is immobilized in the soil forming insoluble compounds not assimilable by plants, and another is washed through a leaching process, which in addition to economic losses, generates a significant problem of environmental pollution.

In the case of phosphorus, in particular, a significant part of the added soluble phosphates is made insoluble by iron and aluminium in acidic soils and by calcium in calcareous soils, progressively forming less assimilable forms. As a result of the various retention mechanisms, most of the phosphorus applied by fertilisation cannot be used by the crops and is retained in the soil in an insoluble form. Given this phenomenon and the cyclical application of fertilisers, the concentration of phosphorus in the soil has increased significantly, so that in many soils long-term harvests could be established if these reserves could be exploited economically. The addition of excess inorganic fertilisers can also lead to environmental problems such as groundwater pollution and eutrophication of waterways. Therefore, it is of great interest to investigate management strategies that are capable of improving the efficiency of phosphorus fertilisation, increasing crop yields, and reducing environmental pollution caused by soil phosphorus loss (Alori et al., 2017).

The importance of microorganisms in the nutrient cycle in soil and their role in plant nutrition is well known. Their active participation in the decomposition and mineralization of organic matter, as well as in the fixation and release of nutrients from the soil, is crucial for the maintenance of plant productivity. The interactions that are established between soil microorganisms and plant roots satisfy important nutritional requirements for both. The roots are directly influenced by the composition and density of the microbial community that develops in them. This is known as the "Rhizosphere Effect", and depends particularly on the plant and its physiological maturity. The practice of inoculating plants with microorganisms is well known in the prior art (Vessey 2003).

In nature there are numerous rhizosphere microorganisms capable of releasing phosphorus from the soil through solubilisation and mineralization (Bhattacharyya and Jha, 2012). This group of microorganisms is known as phosphorus solubilising microorganisms (PSM). Many species of soil fungi and bacteria can solubilise phosphorus in vitro, and some of them increase the bioavailability of insoluble phosphorus in soil for use by plants (Zhu etal., 2011). They solubilise insoluble inorganic (mineral) phosphorus and mineralize insoluble organic phosphorus (Sharma et al., 2013).

One group of microorganisms that has a remarkable importance in this phenomenon is the one involved in the solubilisation of phosphorus from sources that would otherwise be inaccessible to plants.

Phosphate solubilising microorganisms have been isolated in virtually all the soils tested, although the number and proportion of these vary according to the type of soil, climate, and other factors such as the historical evolution of the soil. Many microorganisms are able to assimilate insoluble phosphorus from the soil, releasing a part of it in the form of soluble phosphates that in turn can be used by plants, thus contributing to plant nutrition. It is generally accepted that the solubilisation of phosphates in soil is due to the production of organic acids and chelating oxoacids from sugars (Seshachala and Tallapragada, 2012).

Methods have been described in which phosphate-solubilising microorganisms have been used in fertilisation (Yu et al., 2011).

The methods used in the isolation of phosphate-solubilising microorganisms are mainly based on the use of insoluble inorganic phosphorus salts, such as hydroxyapatite and tricalcium phosphate, in agarized culture media. For the use of these salts, the microorganism produces organic acids that, when diffused in the medium, cause a decrease in pH and as a consequence the formation of a halo of transparency in the vicinity of the colony. However, it has been pointed out that agarized culture media do not always detect the most suitable microorganisms for the solubilisation of phosphates. An alternative scheme and selection of this type of microorganisms has been proposed using stirred liquid cultures as an alternative, noting the importance of certain components of the medium and their concentration in the results achieved and proposing a culture medium in which to carry out the selection (Nautiyal 1999). Other authors, working with agarized media, concluded that the pH buffering capacity of the culture medium used in the isolation is of decisive importance in the selection of strains that produce sufficient amounts of organic acids to present a real interest in biofertilisation. They described that gluconic acid is the main component of the organic acids produced by *Enterobacter asburiae* and that the enzyme glucose dehydrogenase, responsible for the production of gluconic acid, is regulated by starvation of phosphates, that is, that low concentrations of soluble phosphates stimulate the production of acids in this type of microorganism (Gyaneshwar et al., 1999).

The genera *Enterobacter* and *Pantoea* have been used in agriculture as phosphate solubilisers and for protection against plant diseases (Beltrán-Pineda, 2014). *Pantoea dispersa* is a species with genes that encode the enzyme for the detoxification of albicidin. These genes and enzymes have been used to obtain transgenic plants resistant to fungal diseases, which demonstrates their ability to protect against fungal plant diseases. There are several prior art documents that point to *Pantoea dispersa* as a phosphate-solubilising species (Fernandez et al., 2008).

Another aspect that is very important in practice is the use of atmospheric nitrogen-fixing microorganisms in the rhizosphere. Numerous microorganisms have been used for this function, including bacteria of genera such as *Rhizobium, Azotobacter* and *Azospirillum,* and fungi of the genera *Saccharomyces, Hansenula* and *Aspergillus,* among others.

Nitrogen is an abundant element, making up almost 80% of the Earth's atmosphere and a very scarce nutrient. The paradox is easily resolved: atmospheric nitrogen is inert and cannot be used by most organisms, and can only be incorporated into biological synthesis when it has been "fixed" or combined with certain elements such as hydrogen or oxygen. Bacteria are able to fix 1.5 x 10⁸ metric tons per year, an important part of which is synthesized by the Haber-Bosch process.

Experiments carried out in Brazil determined the significant contribution of the N₂ fixed for plants by different microorganisms, with *Azospirillum* being among the main genera. Subsequent quantification studies of the biological nitrogen fixation (BNF) in sugarcane in this same country showed that almost 65% of the total accumulated N₂ was derived from BNF, representing about 150 kg N₂ x ha⁻¹ x year⁻¹, which led to the recommendation to minimize the use of nitrogen fertilisers.

It is known that different species of plants produce different effects on the rhizosphere, and that the strains isolated in one type of plant species have a totally different effect on the rhizosphere from which it was isolated and in different crops. However, *Azospirillum brasilense* is able to colonize plants of different species by adhering to the roots. There are works that have demonstrated the ubiquity of the genus *Azospirillum* carrying out its isolation in varied crops, as well as in regions with very dissimilar climates (Fukami et al., 2016).

It has been described that by inoculating different crops of plants with species of the genus *Azospirillum,* changes in the morphology of the roots appeared and it has been determined that, in the first three weeks after germination, the number of root hairs and branches increases with the inoculation, which produces an increase in the absorption surface of the roots.

In studies of inoculation of wheat plants with *Azospirillum brasilense,* it has been observed that small cell aggregates were formed on the surface of the inoculated roots, and when making cuts, it has been found that some bacteria penetrated the young cells of the roots, but there were many more in the intercellular spaces, as well as in the epidemic layers of the cells. It has been found, in turn, that the adsorption of this bacterium to the roots is weak and is carried out by bacterial-dependent metabolism processes, mediated by recognition factors. Also described is the ability of this species to move in the soil to colonize the roots of the plants and the ability to colonize different crops in addition to cereals, producing morphological changes on their roots analogous to those that occurred in the family *Graminaceae.*

The main element involved in the *Azospirillum*-plant relationship is not only nitrogen, as phosphorus and potassium play a fundamental role in this relationship. Depending on the strain used, there is a quantitative change in the intake of minerals by the plant, significantly increasing some harvests. According to the "theory of multiple mechanisms", the bacterium acts with a pattern of cumulative or sequential effects as a result of mechanisms that occur simultaneously or consecutively (Bashan and De-Bashan 2010).

Nowadays, the use of this genus in the production of biological fertilisers is very common.

It should be noted that many authors agree that the beneficial effects produced by inoculation with microorganisms on plant growth are not only due to the solubilisation of phosphates or the biological fixation of nitrogen. There are mechanisms such as the production of phytohormones and siderophores or the activity of the enzyme 1-aminocyclopropane 1-carboxylate deaminase, among others, which contribute significantly to this effect (Fukami et al., 2018).

Among the microbial relationships that take place in the rhizosphere are the calls for cooperation that are established between the microbial groups that carry out complementary metabolisms. This may be the case for some phosphate-solubilising bacteria that excrete organic acids into the environment, which in turn constitute the main source of carbon in some genera of nitrofixing bacteria, allowing the coexistence of both groups, as well as having a two-fold effect on the improvement of crops and protection against diseases.

The associative development of the genera *Azospirillum* and *Pantoea* has been carried out successfully in the biological fixation of nitrogen, demonstrating that there are no incompatibilities between the two genera and that, due to their different metabolic regulation mechanisms, they can perform different functions in a given ecosystem (Flores et al., 2010; Del Amor y Cuadra, 2011; Schoebitz et al., 2014; Mengual et al., 2014). Mixed cultures of soil microorganisms with different metabolic capacities can develop cooperative relationships with each other and with plants that make nutrient adsorption by them more efficient and can also produce substances that stimulate plant growth, increasing crop productivity and protecting them against pathogenic microorganisms.

The practice of inoculating plants with microorganisms using mixed cultures (also called consortia) that enhance phenomena such as increasing the efficiency of phosphorus absorption by the roots, biological nitrogen fixation, stimulation of plant growth by the production of substances regulating plant growth, as well as siderophores and protection against diseases produced by pathogenic microorganisms among others, has been shown to be the most effective in biofertilisation (Nuti and Giovannetti, 2015).

The physical form of a biofertiliser is a determining factor in the practical outcome provided by that biofertiliser. The viability of organisms present in a biofertiliser during production, formulation, storage, transport/distribution, and field application is directly related to plant growth potential. This limits its use due to compatibility, stability, and survival problems in different soil conditions. Thus, an improved shelf life could be the key to further widespread application of biofertilisers (Brar et al., 2012).

In a biological fertiliser, microorganisms must remain viable, dormant or metabolically active. This factor has two determining aspects: the durability of the product and its ability to colonize the roots of the crops to be stimulated or protected, once applied in the field. The use of free cell preparations is a common practice in agricultural biotechnology and is very effective when working with microorganisms capable of forming resistance structures, as in the case of bacteria of the genus *Bacillus.* This problem is of a different nature when it comes to cells that do not have the capacity to form resistance structures, since crops lose viability over time, and their ability to survive in the soil is very low. Many of the failures that have occurred in the field of biofertilisation and bioprotection are related to this phenomenon. The inoculation of soils with microorganisms capable of promoting the productivity of plants is a very complex process in which numerous factors can have a determining effect. For this reason, it is essential that the biological fertiliser is able to preserve cell viability under adverse conditions for long periods of time and to guarantee as far as possible the colonization capacity of the roots once applied in the field. One of the most important criteria to take into account for this is to obtain biological fertiliser preparations that release an appreciable number of viable cells consistently (Pindi and Satyanarayana, 2012).

In common practice, to increase the lifetime of the biofertiliser formulation, a carrier or a mixture of such carrier materials is selected based on the viability of the microorganisms mixed with them. Similarly, nutrient enrichment is the other strategy for improving shelf life by allowing the microorganism to remain and grow in a non-competitive microenvironment (Yardin et al., 2000).

ES2234417A1 discloses a biological fertiliser comprising bacterial strains of *Azospirillum brasilense* and *Pantoea dispersa* immobilized on a solid support. In particular, said document describes the strain *Azospirillum brasilense* CECT 5802 and the strain *Pantoea dispersa* CECT 5801.

Document WO2009027544A1 discloses a biological fertiliser comprising a solid support in which the bacterial strains *Azospirillum brasilense* CECT 5802 and *Pantoea dispersa* CECT 5801 and, additionally, indole-3-acetic acid or an indole-3-acetic acid promoter have been immobilized.

The change from a biofertiliser comprising microorganisms immobilized on a solid support to a biofertiliser in liquid form is associated with a significant reduction in the viability of such microorganisms and a decrease in the period in which the product remains viable while maintaining efficacy. The shelf life of a liquid biofertiliser is a major concern (Brar et al., 2012).

However, liquid biofertilisers have advantages over biofertilisers in which the microorganisms contained in them are immobilized in a solid support, such as improvements in the mode of application, and can be applied by drip irrigation, spray or by aerial means. It is not possible to apply immobilized biofertilisers on a solid support by drip irrigation or by liquid spraying. Moreover, liquid biofertilisers can be applied to parts of the plants other than the roots, since they can be sprayed on the aerial parts of the plants (stems, leaves, fruits, flowers, etc.). Unlike biofertilisers based on solid carriers, liquid formulations allow including a sufficient amount of nutrients, cell protectors and inducers of cell formation to ensure a long shelf life.

In order to make the production of a biofertiliser economically attractive, it is necessary to make an appropriate selection of the environment in which its production is to be carried out. For this it is essential to carefully select the raw materials and materials to be used, so that costs are minimized. For this purpose, it is necessary that the raw materials and their concentration in the fermentation medium allow to obtain a high cellular concentration or of the desired product at a minimum cost. Products of natural origin are very suitable for this purpose, given their low cost and their harmlessness to the environment. As an additional advantage, these can stimulate desirable effects if they are included in the final preparation. Tomato concentrates have been used in the production of gibberellins, as a source of nitrogen and as a source of carbon for the growth and production of metabolites of microbial origin due to their high content of organic carbon. In addition, collagen hydrolysate used in agriculture is a very suitable and cheap source of organic nitrogen, even for microorganisms unable to produce proteolytic enzymes. The combination of these two sources offers a universal medium of culture, with a very low cost and therefore very attractive for the realization of fermentation processes on a large scale. In addition, its natural origin allows a more ecological agriculture, which society currently demands.

In view of the above, there is an interest for the development of a liquid biofertiliser comprising bacterial strains, in which said bacterial strains maintain their viability for a long time while maintaining their effectiveness, thus obtaining the aforementioned advantages that liquid biofertilisers provide compared to biofertilisers immobilized in solid supports.

### SUBJECT OF THE INVENTION

The object of the invention is a product for biological fertilisation consisting of a liquid formulation containing two strains of bacteria of the genera *Azospirillum* and *Pantoea,* capable of fixing atmospheric nitrogen, solubilising phosphates as well as other mineral nutrients from the soil, and producing high amounts of plant growth stimulating substances. These microorganisms have been formulated in liquid form, which guarantees easy application and high stability in cell viability and provides sufficient organic nutrients and minerals to facilitate the colonization of plant roots. Also an object of the invention is the method for stimulating plant growth comprising the application of the liquid biofertiliser of the invention to said plant, a method for obtaining the liquid biofertiliser of the invention, and a liquid biofertiliser obtainable according to said method.

### DESCRIPTION OF THE INVENTION

The present invention relates to a liquid biofertiliser comprising a strain of *Azospirillum Brasilense* deposited in the Spanish Type Culture Collection (CECT) with deposit number CECT 5802, a strain of *Pantoea dispersa* deposited in the CECT with deposit number CECT 5801, at least one inorganic salt, and a soluble concentrate of molasses.

The present invention differs from the state of the art in that it is a biofertiliser formulated in liquid form, preferably in an aqueous medium suitable for maintaining the viability of the strains of the biofertiliser, maintaining from 60% to 70% of cell viability of both strains after one year of conservation, at temperatures not exceeding 30°C.

Such a suitable aqueous medium is responsible for maintaining the viability of the strains in the liquid formulation. Without such a suitable aqueous medium, the strains would lose their viability in a very short time.

The present invention provides such an aqueous medium suitable for maintaining the strains viable in the liquid biofertiliser. Said liquid medium must comprise at least one inorganic salt and a soluble concentrate of molasses to maintain the viability of the strains of the biofertiliser for a prolonged period of time. Said liquid medium may comprise pH regulating agents and one or more antifoaming agents.

The application dose is significantly reduced in the liquid biofertiliser of the invention comprising the bacterial strains *Azospirillum brasilense* CECT 5802 and *Pantoea dispersa* CECT 5801 with respect to a biofertiliser comprising the same strains immobilized in a solid support, because said liquid biofertiliser can make the bacteria available to the plants more quickly than in the form immobilized in a solid support. This dose reduction is approximately 20-fold, from an application of immobilized biofertiliser on a solid support of 300 kg/ha to an application of said liquid biofertiliser of 15 L/ha. Additionally, the bacterial strains present in the liquid biofertiliser of the invention remain viable, in said liquid biofertiliser, for at least one year while maintaining their effectiveness.

The complex technical problem to be solved would therefore be to provide a biofertiliser comprising bacterial strains, formulated in liquid form, which allows its application in plants with a reduced dose and in which said bacterial strains remain viable for at least one year, maintaining their effectiveness.

This invention, defined by the object of the claims, provides a solution to said technical problem.

The advantages of such a liquid biofertiliser over biofertilisers immobilized on a solid support are based on the liquid formulation form itself. Said liquid biofertiliser, being a liquid product in which the bacteria remain viable for at least one year, aids in the mode of application of the same, as it can be applied by drip irrigation, spraying or by aerial means. Application by drip irrigation and liquid spraying is not possible with biofertilisers immobilized on a solid support.

In a preferred embodiment, said biofertiliser is an aqueous suspension.

In another preferred embodiment, said biofertiliser has a pH from 6 to 8. Suitable pH regulating agents are used for this purpose.

In another preferred embodiment, said biofertiliser further comprises at least one pH regulating agent and at least one antifoaming agent. Still more preferably, said biofertiliser comprises the pH-regulating agents malic acid and KOH. Still more preferably, said defoaming agent is polypropylene glycol.

In a more preferred embodiment, said inorganic salt is selected from the group consisting of (NH₄)₂SO₄, K₂HPO₄, ZnSO₄, MnSO₄, CuSO₄, MgSO₄ and CaCl₂. In a still more preferred embodiment, said biofertiliser comprises the inorganic salts (NH₄)₂SO₄, K₂HPO₄, ZnSO₄, MnSO₄, CuSO₄, MgSO₄ and CaCl₂.

The inorganic salts and soluble concentrate of molasses are ingredients used as nutrient sources in the liquid culture medium in which the strains of the invention grow, necessary to maintain the viability of the microorganisms present in the biofertiliser.

In another preferred embodiment, said pH regulating agents are malic acid and KOH.

In another preferred embodiment, said biofertiliser of the invention comprises (NH₄)₂SO₄, K₂HPO₄, ZnSO₄, MnSO₄, CuSO₄, MgSO₄, CaCl₂, soluble concentrate of molasses, malic acid, KOH and polypropylene glycol. More preferably, said biofertiliser of the invention comprises (NH₄)₂SO₄ 0.5-3.5% (w/w), K₂HPO₄ 0.1-1% (w/w), ZnSO₄▪7H₂O 0.005-0.1% (w/w), MnSO₄▪H₂O 0.005-0.1% (w/w), CuSO₄▪5H₂O 0.001-0.01% (w/w), MgSO₄▪7H₂O 0.001-0.01% (w/w), CaCl₂▪2H₂O 0.001-0.01% (w/w), soluble concentrate of molasses 0.05-2% (w/w), malic acid 0.01-0.1% (w/w), KOH 0.005-1% (w/w) and polypropylene glycol 0.001-0.1% (w/w). Still more preferably, said biofertiliser of the invention comprises (NH₄)₂SO₄ 1.5-2.5% (w/w), K₂HPO₄ 0.18-0.20% (w/w), ZnSO₄▪7H₂O 0.01-0.02% (w/w), MnSO₄▪H₂O 0.01-0.02% (w/w), CuSO₄▪5H₂O 0.001-0.002% (w/w), MgSO₄▪7H₂O 0.003-0.004% (w/w), CaCl₂▪2H₂O 0.001-0.002% (w/w), soluble concentrate of molasses 0.25-0.75% (w/w), malic acid 0.035-0.045% (w/w), KOH 0.02-0.1% (w/w) and polypropylene glycol 0.005-0.01% (w/w).

In another preferred embodiment, said biofertiliser further comprises KH₂PO₄ and/or NH₄Cl.

In another preferred embodiment, said fertiliser further comprises one or more ingredients selected from the group consisting of tomato paste, collagen hydrolysate, and yeast extract. In yet another preferred embodiment, said biofertiliser comprises tomato paste, collagen hydrolysate, and yeast extract.

Tomato paste is a natural product used as a carbon source in the liquid culture medium in which the strains of the invention grow.

Collagen hydrolysate is a natural product used as a source of nitrogen in the liquid culture medium in which the strains of the invention grow.

Natural yeast extract is a natural product rich in vitamins, especially B complex, amino acids, and other growth factors. It is used as a source of nutrients in the liquid culture medium in which the strains of the invention grow.

In another preferred embodiment, said biofertiliser further comprises KH₂PO₄, NH₄Cl, tomato paste, collagen hydrolysate, and yeast extract. More preferably, said biofertiliser comprises KH₂PO₄ 0.05-0.3% (w/w), NH₄Cl 0.01-2% (w/w), tomato paste 0.5-3% (w/w), collagen hydrolysate 0.01-2% (w/w) and yeast extract 0.01-2% (w/w).

The present invention also relates to a method for stimulating the growth of a plant, comprising applying said liquid biofertiliser to said plant.

In a preferred embodiment of said method for stimulating plant growth, the application of said liquid biofertiliser is selected from the group consisting of fertigation, spray, and drip irrigation.

In another preferred embodiment of said method for stimulating plant growth, the application dose of said liquid biofertiliser is from 5 to 30 L/ha. In a more preferred embodiment of said method for stimulating plant growth, said application dose is from 10 to 20 L/ha.

In another preferred embodiment of said method for stimulating plant growth, said plant is selected from the group consisting of lettuce, broccoli, and tomato.

The present invention also relates to a method for obtaining a liquid biofertiliser, comprising:
(a) culturing a strain of *Azospirillum Brasilense* deposited in the Spanish Type Culture Collection (CECT) with deposit number CECT 5802 and a strain of *Pantoea dispersa* deposited in the CECT with deposit number CECT 5801 in separate and appropriate first liquid fermentation media for each strain, at a temperature from 25°C to 35°C, under stirring and aeration and
(b) adding in cold the fermentation broths obtained in the previous step to a second fresh liquid medium, previously sterilized.

In a preferred embodiment of said method for obtaining the biofertiliser of the invention, said biofertiliser is aqueous, more specifically an aqueous suspension.

In another preferred embodiment of said method for obtaining the biofertiliser of the invention, the first liquid fermentation media comprise at least one of the ingredients selected from the group consisting of tomato paste, collagen hydrolysate, and yeast extract. In another more preferred embodiment of said method for obtaining, the first liquid fermentation media comprise tomato paste, collagen hydrolysate, and yeast extract. The addition of these naturally occurring products is particularly appropriate for the use of these biofertilisers in organic farming.

In another preferred embodiment of said method for obtaining the biofertiliser of the invention, the first liquid fermentation media further comprise at least one inorganic salt selected from the group consisting of K₂HPO₄, KH₂PO₄, and NH₄Cl. In another more preferred embodiment of said method for obtaining the biofertiliser of the invention, the first liquid fermentation media comprise the inorganic salts K₂HPO₄, KH₂PO₄ and NH₄Cl.

In another embodiment of said method for obtaining the biofertiliser of the invention, the first liquid fermentation media comprise K₂HPO₄, KH₂PO₄, NH₄Cl, tomato paste, collagen hydrolysate and yeast extract. More preferably, the first liquid fermentation media comprise K₂HPO₄ 1-6% (w/w), KH₂PO₄ 0.01-0.6% (w/w), NH₄Cl 0.02-4% (w/w), tomato paste 1-6% (w/w), collagen hydrolysate 0.02-4% (w/w) and yeast extract 0.02-4% (w/w).

In another embodiment of such a method for obtaining the biofertiliser of the invention, the second liquid fresh medium comprises at least one inorganic salt, a soluble concentrate of molasses, at least one pH-regulating agent, and at least one antifoaming agent.

In a more preferred embodiment of said method for obtaining the biofertiliser of the invention, said inorganic salt is selected from the group consisting of (NH₄)₂SO₄, K₂HPO₄, ZnSO₄, MnSO₄, CuSO₄, MgSO₄ and CaCl₂. In a more preferred embodiment of the method for obtaining the biofertiliser of the invention, the second liquid fresh medium comprises the inorganic salts (NH₄)₂SO₄, K₂HPO₄, ZnSO₄, MnSO₄, CuSO₄, MgSO₄ and CaCl₂.

In another preferred embodiment of said method for obtaining the biofertiliser of the invention, the second fresh liquid medium comprises the pH-regulating agents malic acid and KOH.

In another preferred embodiment of said method for obtaining the biofertiliser of the invention, said antifoaming agent is polypropylene glycol.

In another preferred embodiment of said method for obtaining the biofertiliser of the invention, the second fresh liquid medium comprises (NH₄)₂SO₄, K₂HPO₄, ZnSO₄, MnSO₄, CuSO₄, MgSO₄, CaCl₂, a soluble concentrate of molasses, malic acid, KOH and polypropylene glycol. More preferably, the second fresh liquid medium comprises (NH₄)₂SO₄ 1-7% (w/w), K₂HPO₄ 0.001-0.2% (w/w), ZnSO₄▪7H₂O 0.001-0.2% (w/w), MnSO₄▪H₂O 0.001-0.2% (w/w), CuSO₄▪5H₂O 0.001-0.02% (w/w), MgSO₄▪7H₂O 0.001-0.02% (w/w), CaCl₂▪2H₂O 0.001-0.02% (w/w), soluble concentrate of molasses 0.1-2% (w/w), malic acid 0.02-0.2% (w/w), KOH 0.01-2% (w/w) and polypropylene glycol 0.001-0.2% (w/w). More preferably, the second fresh liquid medium comprises (NH₄)₂SO₄ 3-5% (w/w), K₂HPO₄ 0.04-0.06% (w/w), ZnSO₄▪7H₂O 0.02-0.04% (w/w), MnSO₄▪H₂O 0.02-0.04% (w/w), CuSO₄▪5H₂O 0.002-0.004% (w/w), MgSO₄▪7H₂O 0.006-0.008% (w/w), CaCl₂▪2H₂O 0.002-0.004% (w/w), soluble concentrate of molasses 0.5-1.5% (w/w), malic acid 0.07-0.09% (w/w), KOH 0.04-0.2% (w/w) and polypropylene glycol 0.01-0.02% (w/w).

In another preferred embodiment of said method for obtaining the biofertiliser of the invention, from 20% to 30% (w/w) of the fermentation broths obtained in step (a) are added to said second fresh liquid medium.

In another preferred embodiment of said method for obtaining the biofertiliser of the invention, the cultures of step (a) are carried out for a period of time from 8 to 20 hours.

In another preferred embodiment of said method for obtaining the biofertiliser of the invention, the stirring rate in step (a) is from 400 to 800 r.p.m.

In another preferred embodiment of said method for obtaining the biofertiliser of the invention, the aeration in step (a) is from 0.5 to 2 L/min.

The present invention also relates to a liquid biofertiliser obtainable according to said method for obtaining the invention.

Field tests have been carried out under outdoor and greenhouse production conditions with this liquid biofertiliser, with very satisfactory results. Such field trials are described in examples 3-5.

Unless defined otherwise, all the technical and scientific terms used herein have the same meaning as those customarily understood by a person skilled in the field of the invention. Methods and materials that are similar and equivalent to those described herein may be used in the practice of the present invention.

Throughout the description and the claims, the term "comprising", "that comprises" and their variants are meant in a non-limiting sense and therefore should not exclude other technical features. The term "comprises", "comprising" and its variants, throughout the description and claims, specifically includes the term "consists of", "consisting of" and their variants.

### Definitions

Herein, the term "viability" refers to that the microorganisms of the liquid biofertiliser maintain their ability to reproduce, to colonize the roots and thus maintain their ability to exercise their biofertilising function. This viability means that the biofertiliser is stable and functional during storage under appropriate conditions. In particular, both strains of the liquid biofertiliser maintain from 60% to 70% cell viability after one year of storage, at temperatures not exceeding 30°C. The terms "feasibility," "viable," and the like have, for purposes of this specification, the same meaning as the terms "stability," "stable," and the like.

The term "aqueous liquid biofertiliser" herein refers to a liquid biofertiliser in which the majority solvent is water. Said aqueous liquid biofertiliser comprises at least one inorganic salt and a soluble concentrate of molasses and may comprise acids, hydroxides, polypropylene glycol and other ingredients. Examples of other ingredients are: tomato paste, collagen hydrolysate and yeast extract. Said liquid biofertiliser may consist of a buffered formulation.

In the present specification, the term "soluble concentrate of molasses" refers to a product obtained from industrial fermentation processes for the production of yeast, alcohol and organic acids on molasses substrate. Molasses are obtained from sugar crystallization processes and are a residue of this process from which no more sugar can be obtained by physical methods. Molasses can be obtained from sugar cane.

The term "pH-regulating agents" herein refers to chemicals that adjust the pH of a solution to a desired value or that are capable of maintaining the pH of a solution within a reduced range. Examples of regulatory agents are carboxylic acids, dicarboxylic acids, malic acid, metal hydroxides, such as KOH, NaOH, etc.

Herein, the term "antifoaming agent" refers to a chemical that prevents the formation of foam in a solution.

In the present specification, the term "collagen hydrolysate" refers to a product obtained from biological animal tissues, such as skin, bones, spines, scales, of porcine, bovine, chicken, fish origin, etc. The collagen present in these tissues is subjected to a hydrolysis process that separates the polypeptide chains from the collagen. These chains are then fragmented into smaller segments, using chemicals (chemical hydrolysis) or proteolytic enzymes (enzymatic hydrolysis). It is a product used in the preparation of culture media for microorganisms as a source of nitrogen.

Herein, the term "yeast extract" refers to a water-soluble extract formed by autolysing yeast cells. It is a product rich in vitamins, especially B complex, amino acids and other growth factors. It is used in the preparation of culture media for microorganisms as a source of nutrients.

Herein, the term "fertigation" refers to a fertiliser application technique that allows for the simultaneous application of water and fertilisers through the irrigation system.

Herein, the term "spraying" refers to a fertiliser application technique that distributes a liquid particulate.

### DESCRIPTION OF EMBODIMENTS

### Example 1. Obtaining the liquid biofertiliser

### Obtaining fermentation broths of the strains

A tomato culture medium was prepared comprising tomato paste as a carbon source, animal skin collagen hydrolysate of fertilising use as an organic nitrogen source, the composition of which is as described in **Table 1.**

The tomato paste was a sterile product obtained from tomatoes (*Solanum Lycopersicum L.*/*Lycopersicum Sculentum "Mill"*) whole, crushed, sieved, homogenized, concentrated, pasteurized and aseptically packaged (commercial product "Concentrate of tomato 28/30 ° Brix" by Messjero Alimentacion, S.L.). This product had 28-30 °Brix, contained 30-32% (w/w) dry extract, pH 4.0-4.4, acidity (citric acid) 1.6-2.2, Bostwick viscosity (20°C) 6-11 cm/30 seconds, 0.6 mm mesh size and a Howard count less than 60%. The average values per 100 g of this product were: energy value 92 Kcal/387 kJ, total fats 0.2 g, saturated fatty acids less than 0.1 g, carbohydrates 22 g, sugars 12 g, dietary fibre 7 g, proteins 5 g and salt less than 0.1 g.

Collagen hydrolysate was a powdered product consisting of a powdered mixture of amino acids and peptides obtained by chemical hydrolysis (commercial product "Protifert P N 142 de Sicit 2000 S.p.A.). The product was an ivory-white powder, containing 95% (w/w) dry matter, total nitrogen 14% (w/w), volumetric mass 300 g/L, pH 6.0-7.5 in 10% (w/w) solution, thermal stability greater than 200°C for oxidation in static air and greater than 300°C in an inert atmosphere.

The yeast extract used is a powder product (commercial product "Yeast Extract Powder" of Oxoid Ltd). The product was a pale beige powder.

**Table 1. Composition of the tomato culture medium**

| Component | 9 |
|---|---|
| Tomato paste | 36.00 |
| K₂HPO₄ | 3.30 |
| KH₂PO₄ | 2.75 |
| NH₄Cl | 0.84 |
| Collagen hydrolysate | 5.00 |
| Yeast extract | 0.75 |
| H₂O (qs) | 1 L |

The pH of the tomato culture medium was 7.0. The tomato culture medium was sterilized at 121°C for 30 minutes.

With the tomato culture medium, high cell counts were achieved in 12-14 hours of fermentation for both strains. The culture medium is very economical and, more importantly, high cell counts of the order of 10⁹-10¹⁰ cells/mL are achieved in both cases.

The purity of an isolate of the *Azospirillum brasilense* CECT 5802 strain was verified by taking an ampoule of said isolate, sowing a sample of said ampoule in Congo Red medium plates and incubating at 30°C for 72 hours. A portion of the culture of this plate was taken with a loop, inoculated in a 1000 mL Erlenmeyer flask, with 100 mL of the tomato culture medium and incubated under stirring at 30°C for 16 hours. After this time, the contents of the flask, which was in an exponential phase, were inoculated in a 3 L Braun Biotech BIOSTAT^{®} B fermenter with 1.9 L of the tomato culture medium. Fermentation was carried out for 16 hours at a stirring speed of 600 r.p.m., an aeration of 2 L/min (1 v.v.m. (volume of air per volume of medium per minute)) and a temperature of 30°C. The pH was allowed to vary freely and reached a value of 6.8. A concentration of 8.9x10⁹ cells/mL was reached. The specific exponential phase growth rate (µ) was 0.25 h⁻¹.

For the strain *Pantoea dispersa* CECT 5801, the same fermentation scheme was followed, with small modifications which are described below. The purity of the strain isolate was tested in MacConkey culture medium, incubating an inoculum of said strain in an orbital shaker for 12 hours. The culture in the BIOSTAT^{®} B fermenter was carried out for 12 hours at a stirring rate of 600 r.p.m., an aeration of 3 L/min (1.5 v.v.m.) and a temperature of 30°C. The pH was allowed to vary freely and reached a value of 6.9. A concentration of 9.5x10⁹ cells/mL was reached. The specific exponential phase growth rate for this strain was µ = 0.57 h⁻¹.

### Preparation of fresh medium

A fresh medium was prepared. The ingredients that make up the fresh medium are described in **Table 2.** The concentration of the ingredients in this example was the middle value of the range indicated in **Table 2.**

Soluble concentrate of molasses was a concentrated liquid product obtained from industrial fermentation processes for the production of yeast, alcohol and organic acids on molasses substrate and other tipping liquors, a source of nitrogen and mineral salts (commercial product "Organic Nitrogen Fertilizer Fluid" of ED&F Man Liquid Products Italia Srl). The product was a thick dark brown or black liquid, had a viscosity of 200-2000 cp at 20°C, specific gravity 1.15-1.35, flash point above 200°C, miscible in water and decomposition temperature above 45°C and pH from 5.5 to 8.5.

The polypropylene glycol was a polymer, a liquid product (commercial product "VORANOL*2000 L Polyol" from The Dow Chemical Company). The product was a colourless liquid, flash point 230°C COC, vapour density (air = 1) greater than 1, specific gravity (water = 1) 1.01, density 1.0 g/cm³, dynamic viscosity 305-335 mPa·s at 25°C.

**Table 2. Components of the fresh medium**

| Component | Concentration (% w/w) |
|---|---|
| (NH₄)₂SO₄ | 3-5% |
| K₂HPO₄ | 0.04-0.06% |
| ZnSO₄▪7H₂O | 0.02-0.04% |
| MnSO₄▪H₂O | 0.02-0.04% |
| CuSO₄▪5H₂O | 0.002-0.004% |
| MgSO₄▪7H₂O | 0.006-0.008% |
| CaCl₂▪2H₂O | 0.002-0.004% |
| Soluble concentrate of molasses | 0.5-1.5% |
| Malic Acid | 0.07-0.09% |
| Polypropylene glycol | 0.01-0.02% |
| KOH | 0.04-0.2% |

A fermentation reactor with stirring and temperature control was loaded with the amount of water needed to obtain the desired final volume of product. This amount of water required was approximately 44% w/w. The stirrer was started and the salts were added, following the order in which said salts appear in **Table 2,** until complete dissolution.

Next, the molasses concentrate, malic acid and finally polypropylene glycol were incorporated, while stirring was maintained.

Once a completely homogeneous mixture was obtained, the pH of the medium was adjusted to 7.00 by the addition of potash (KOH).

Finally, the fresh medium was sterilized at 121°C for 20 minutes.

### Obtaining the liquid biofertiliser

The fresh medium was allowed to cool. Next, the fermentation broths of the strains *Azospirillum brasilense* CECT 5802 (25% w/w) and *Pantoea dispersa* CECT 5801 (25% w/w), obtained according to the procedure described in the section "Obtaining fermentation broths of the strains" of this example, were added. Said fermentation broths were added by slowly stirring until a homogeneous mixture was obtained.

### Example 2. Assessment of the cell viability of the liquid biofertiliser

The cell viability of the liquid biofertiliser was periodically checked. The cell viability of the strains *Azospirillum brasilense* CECT 5802 and *Pantoea dispersa* CECT 5801 was tested in Congo Red medium and MacConkey medium, respectively. The liquid biofertiliser retained more than 60% cell viability after one year of storage, at temperatures not exceeding 30°C, for both strains.

### Example 3. Assay with the liquid biofertiliser on lettuce

This trial was conducted outdoors between November 2018 and February 2018 in Santomera, Murcia.

In some treatments of this example and the following examples the liquid biofertiliser obtained in Example 1 was used.

**Tables 3 and 4** show the production results obtained in the test.

**Table 3. Production of lettuce by Thesis (treatment)**

| | kg/hectare |
|---|---|
| Control without fertiliser | 41,848 |
| 100% chemical fertiliser | 44,928 |
| Liquid biofertiliser 2.5 L/ha | 45,213 |
| Liquid biofertiliser 5 L/ha | 45,422 |
| Liquid biofertiliser 2.5 L/ha + 50% fertilised | 45,742 |

**Table 4. Average weight of lettuce per thesis**

| | Average weight of lettuce (g) | Increase with respect to the control without fertiliser |
|---|---|---|
| Control without fertiliser | 313.9 | -- |
| 100% chemical fertiliser | 337.0 | 7.4 % |
| Liquid biofertiliser 2.5 L/ha | 339.1 | 8.0 % |
| Liquid biofertiliser 5 L/ha | 340.7 | 8.5 % |
| Liquid biofertiliser 2.5 L/ha + 50% fertilised | 343.1 | 9.3 % |

As can be seen in **Tables 3 and 4,** the liquid biofertiliser of the invention, at 2.5 and 5 L/ha, obtains superior yields both with respect to the untreated control and to the thesis with the recommended fertiliser according to the integrated production standards of the Region of Murcia (B.O.R.M., Order 8024).

The liquid biofertiliser of the invention is effective in decreasing the need for chemical fertilisation input since it obtains results similar to or superior to 100% chemical fertilisation.

### Example 4. Assay with the liquid biofertiliser on broccoli

This trial was conducted outdoors between November 2018 and February 2019 in Santomera, Murcia. **Tables 5 and 6** show the production results obtained in the test.

**Table 5. Production of broccoli by Thesis**

| | kg/hectare |
|---|---|
| Control without fertiliser | 14,357 |
| 100% chemical fertiliser | 15,270 |
| Liquid biofertiliser 2.5 L/ha | 15,492 |
| Liquid biofertiliser 5 L/ha | 16,505 |

**Table 6. Average weight of broccoli by thesis**

| | Average weight of broccoli (g) | Increase with respect to the control without fertiliser |
|---|---|---|
| Control without fertiliser | 301.5 | -- |
| 100% chemical fertiliser | 320.6 | 6.4 % |
| Liquid biofertiliser 2.5 L/ha | 325.3 | 7.9 % |
| Liquid biofertiliser 5 L/ha | 346.6 | 15.0 % |

As can be seen in **Tables 5 and 6,** the liquid biofertiliser of the invention, at 2.5 and 5 L/ha, obtains superior yields with respect to the untreated control.

The liquid biofertiliser of the invention is effective in decreasing the need for chemical fertilisation input since it obtains results superior to 100% chemical fertilisation.

### Example 5. Assay with the liquid biofertiliser on tomato

This trial was conducted in a greenhouse between September 2017 and May 2018 in Mazarrón, Murcia. **Table 7** shows the production results obtained in the test.

**Table 7. Production of tomatoes by Thesis**

| | kg/hectare |
|---|---|
| 100% chemical fertiliser | 61,869 |
| Liquid biofertiliser 5 L/ha | 69,993 |
| Liquid biofertiliser 2.5 L/ha + 50% fertilised | 74,511 |

**Table 8. Chemical fertilisers used per treatment**

| | Monopotassium phosphate (Kg) | Potassium nitrate (Kg) | Calcium nitrate (Kg) | Total Kg |
|---|---|---|---|---|
| 100% fertiliser | 157 | 291 | 282 | 730 |
| Liquid biofertiliser 5 L/ha | 0 | 0 | 0 | 0 |
| Liquid biofertiliser 2.5 L/ha + 50% fertilised | 78 | 145 | 141 | 365 |

The liquid biofertiliser of the invention, at 5 L/ha, provides an increase in annual production in kg/ha of 12% in the thesis applied with the liquid biofertiliser of the invention only, without any type of chemical contribution. In the thesis in which 50% of chemical fertilisation is maintained, the increase reaches 20%.

For the treatment with conventional chemical fertiliser, the consumption of fertilising products has risen to 730 kg/ha **(Table 8),** which in the thesis with product alone, without chemical contributions, is totally replaced by only 20 L/ha of liquid biofertiliser.

In the thesis in which half of the usual fertilisation has been maintained, the savings have been 365 kg/ha of fertilisers, achieving a notable production increase of 20%, contributing no more than 10 L/ha of liquid biofertiliser.

### REFERENCE TO THE DEPOSITED BIOLOGICAL MATERIAL

The strains of *Azospirillum brasilense* and *Pantoea dispersa* were deposited on June 9, 2003, according to the Budapest Treaty on the International Recognition of the Deposit of Microorganisms for the Purposes of Patent Procedure, at the International Depository Authority (IDA) Spanish Type Culture Collection (CECT), by the depositor Probelte, S.A. The depositor identified the strains as *"Azospirillum brasilense* M₃" and *"Pantoea dispersa* C₃". After successful completion of the strain viability tests, the IDA accepted the strains, confirmed the date, indicated above, on which the IDA received the deposit and assigned the deposit number CECT 5802 and CECT 5801, respectively, to those strains.

The *Azospirillum brasilense* CECT 5802 strain was isolated from roots of pea plants (*Pisum sativum*) harvested in the Murcia region. The ability of the *Azospirillum brasilense* CECT 5802 strain to stimulate plant grown was tested by laboratory and greenhouse bioassays. The *Azospirillum brasilense* CECT 5802 strain was the one that produced the greatest growth-stimulating effect of the more than 50 nitrogen-fixing bacteria isolates tested. The production of 3-indole acetic acid and other substances that promote plant growth was verified. The presence of other cytokine-type phytohormones was detected. In the production of 3-indole acetic acid, values of more than 95% transformation of tryptophan in tomato medium were achieved with 150 mg x L⁻¹ of this amino acid. The activity of the enzyme 1-aminocyclopropane 1-carboxylate deaminase present in this strain was also verified, through growth in media with 1-aminocyclopropane 1-carboxylic acid (ACC) as the only source of nitrogen.

The *Pantoea dispersa* CECT 5801 strain was isolated from the rhizosphere of *Shorgum halepense* plants collected in the region of Murcia. The characterization of the organic acids produced by the *Pantoea dispersa* CECT 5801 strain was carried out, and it was found that it mainly produces gluconic acid, as well as other organic acids in small quantities. The selection was made by its ability to stimulate plant growth and to produce auxins. Its ability to produce siderophores was determined. The ability to stimulate plant growth was tested by laboratory and greenhouse bioassays. The activity of the enzyme 1-aminocyclopropane 1-carboxylate deaminase present in this strain was also verified, through growth in media with 1-aminocyclopropane 1-carboxylic acid (ACC) as the only source of nitrogen.

### REFERENCES

Alori, E.T., Glick, B.R. & Babalola, O.O. (2017). Microbial Phosphorus Solubilization and Its Potential for Use in Sustainable Agriculture. Front. Microbiol. 8:971.
Bashan, Y. & de-Bashan, L.E. (2010). How the plant growth-promoting bacterium Azospirillum promotes plant growth-a critical assessment. Adv Agron 108:77-136.
Beltrán-Pineda, M.E. (2014). The solubilisation of phosphates as a microbial strategy to promote plant growth. Corpoica Ciencia y Tecnología Agropecuaria, Vol. 15, No. 1, 101-113.
Bhattacharyya, P. N., & Jha, D. K. (2012). Plant growth-promoting rhizobacteria (PGPR): emergence in agriculture. World J. Microbiol. Biotechnol. 28, 1327-1350.
Brar, S.K., Sarma, S.J. & Chaabouni, E. (2012). Shelf-life of Biofertilisers: An Accord between Formulations and Genetics. J Biofertil Biopestici, 3:5.
Del Amor, F.M. & Cuadra, P. (2011). Plant growth-promoting bacteria as a tool to improve salinity tolerance in sweet peppers. Functional Plant Biology 39(1): 82-90.
Fernández, A.I., Villaverde, M., Nicolas, J.A., García-Gómez, A. & Malo, J. (2008). Scattered pantoea; Plant Growth Promoting Rhizobacteria (PGPR). 7th EEAS Congress*.*
Flores, P., Fenoll, J., Hellín, P. & Aparicio-Tejo, P. 2010. Isotopic evidence of significant assimilation of atmospheric-derived nitrogen fixed by Azospirillum brasilense co-inoculated with phosphate-solubilising Pantoea dispersed in pepper seedling. Applied Soil Ecology, 46: 335-340.
Fukami, J., Nogueira, M.A., Araujo, R.S. & Hungary, M. (2016). Accessing inoculation methods of maize and wheat with Brazilian Azospirillum. AMB Express, 6:1.
Fukami, J., Cerezini, P. & Hungria, M. (2018). Azospirillum: benefits that go far beyond biological nitrogen fixation AMB Expr, 8:73.
Gyaneshwar, P., Parekh, L.J., Archana, G., Poole, P.S., Collins, M.D., Huston, R.A. & Kumar, G.N. (1999). Involvement of a phosphate starvation inducible glucose dehydrogenase in soil phosphate solubilisation by Enterobacter asburiae. FEMS Microbiology Letters 171: 223-229.
Mengual, C., Roldán, A., Caravaca, F. & Schoebitz. M. 2014. Advantages of inoculation with immobilized rhizobacteria versus modification with olive-mill waste in the afforestation of a semiarid area with Pinus halepensis Mill. Ecological Engineering, 73: 1-8.
Nautiyal, C. S. (1999). An efficient microbiological growth medium for screening phosphate solubilising microorganisms. FEMS Microbiology Letters Vol 170, 1: 265-270.
Nuti, M. & Giovannetti, G. (2015). Borderline Products between Bio-fertilisers/Bio-effectors and Plant Protectants: The Role of Microbial Consortium. Journal of Agricultural Science and Technology, A 5: 305-315.
Pindi, P.K. & Satyanarayana, S.D.V. (2012) Liquid Microbial Consortium-A Potential Tool for Sustainable Soil Health. J Biofertil Biopestici, 3:4
Schoebitz, M., Mengual, C. & Roldán, A. (2014). Combined effects of clay immobilized Azospirillum brasilense and Pantoea dispersa and organic olive residue on plant performance and soil properties in the revegetation semiarid area. Science of the Total Environment. 466-467: 67-73.
Seshachala, U. & Tallapragada, P. (2012). Phosphate solubilisers from the rhizosphere of Piper nigrum L. in Karnataka, India. Chil. J. Agric. Res. 72, 397-403.
Sharma, S. B., Sayyed, R. Z., Trivedi, M. H. & Gobi, T. A. (2013). Phosphate solubilising microbes: sustainable approach for managing phosphorus deficiency in agricultural soils. Springerplus 2, 587-600.
Vessey, J.K. (2003). Plant growth promoting rhizobacteria as biofertilisers. Plant and Soil, 255: 571-586.
Yardin, M.R., Kennedy, I.R. & Thies, J.E. (2000). Development of high quality carrier materials for field delivery of key microorganisms used as bio-fertilisers and bio-pesticides. Radiation Physics and Chemistry 57: 565-568.
Yu, X., Liu, X., Hui, T.Z., Liu, G.H. & Mao, C. (2011). Isolation and characterization of phosphate solubilising bacteria from walnut and their effect on growth and phosphorus mobilization. Biol Fertil Soils 47:437-446.
Zhu, F., Qu, L., Hong, X., & Sun, X. (2011). Isolation and characterization of a phosphate solubilising halophilic bacterium Kushneria sp. YCWA18 from Daqiao Saltern on the coast of yellow sea of China. Evid. Based Complement. Alternate. Med. 615032.

## Claims

1. A liquid biofertiliser comprising a strain of *Azospirillum brasilense* deposited in the Spanish Type Culture Collection (CECT) with deposit number CECT 5802, a strain of *Pantoea dispersa* deposited in the CECT with deposit number CECT 5801, at least one inorganic salt and a soluble concentrate of molasses.

2. The biofertiliser according to claim 1, wherein the biofertiliser is an aqueous suspension.

3. The biofertiliser according to claim 1 or 2, wherein said biofertiliser has a pH from 6 to 8.

4. The biofertiliser according to any one of claims 1-3, further comprising at least one pH-regulating agent and at least one antifoaming agent

5. The biofertiliser according to any one of claims 1-4, wherein said inorganic salt is selected from the group consisting of (NH₄)₂SO₄, K₂HPO₄, ZnSO₄, MnSO₄, CuSO₄, MgSO₄, and CaCl₂.

6. The biofertiliser according to claim 5, comprising the inorganic salts (NH₄)₂SO₄, K₂HPO₄, ZnSO₄, MnSO₄, CuSO₄, MgSO₄ and CaCl₂.

7. The biofertiliser according to any one of claims 4-6, comprising the pH-regulating agents malic acid and KOH.

8. The biofertiliser according to any one of claims 4-7, wherein said antifoaming agent is polypropylene glycol.

9. The biofertiliser according to any one of claims 1-8, comprising (NH₄)₂SO₄, K₂HPO₄, ZnSO₄, MnSO₄, CuSO₄, MgSO₄, CaCl₂, soluble concentrate of molasses, malic acid, KOH and polypropylene glycol.

10. The biofertiliser according to claim 9, comprising (NH₄)₂SO₄ 0.5-3.5% (w/w), K₂HPO₄ 0.1-1% (w/w), ZnSO₄▪7H₂O 0.005-0.1% (w/w), MnSO₄▪H₂O 0.005-0.1% (w/w), CuSO₄▪5H₂O 0.001-0.01% (w/w), MgSO₄▪7H₂O 0.001-0.01% (w/w), CaCl₂▪2H₂O 0.001-0.01% (w/w), soluble concentrate of molasses 0.05-2% (w/w), malic acid 0.01-0.1% (w/w), KOH 0.005-1% (w/w), and polypropylene glycol 0.001-0.1% (w/w).

11. The biofertiliser according to claim 10, comprising (NH₄)₂SO₄ 1.5-2.5% (w/w), K₂HPO₄ 0.18-0.20% (w/w), ZnSO₄▪7H₂O 0.01-0.02% (w/w), MnSO₄▪H₂O 0.01-0.02% (w/w), CuSO₄▪5H₂O 0.001-0.002% (w/w), MgSO₄▪7H₂O 0.003-0.004% (w/w), CaCl₂▪2H₂O 0.001-0.002% (w/w), soluble concentrate of molasses 0.25-0.75% (w/w), malic acid 0.035-0.045% (w/w), KOH 0.02-0.1% (w/w) and polypropylene glycol 0.005-0.01% (w/w).

12. The biofertiliser according to any one of claims 1-11, further comprising KH₂PO₄ and/or NH₄Cl.

13. The biofertiliser according to any one of claims 1-12, further comprising one or more ingredients selected from the group consisting of tomato paste, collagen hydrolysate and yeast extract.

14. The biofertiliser according to any one of claims 1-13, further comprising tomato paste, collagen hydrolysate and yeast extract.

15. The biofertiliser according to any one of claims 1-14, further comprising KH₂PO₄, NH₄Cl, tomato paste, collagen hydrolysate and yeast extract.

16. The biofertiliser according to claim 15, comprising KH₂PO₄ 0.05-0.3% (p/p), NH₄Cl 0.01-2% (p/p), tomato paste 0.5-3% (w/w), collagen hydrolysate 0.01-2% (w/w), and yeast extract 0.01-2% (w/w).

17. A method for stimulating plant growth, comprising applying to said plant a liquid biofertiliser according to any one of claims 1-16.

18. The method according to claim 17, wherein the application of said liquid biofertiliser is selected from the group consisting of fertigation, spraying, and drip irrigation.

19. The method according to claim 17 or 18, wherein the application dose of said liquid biofertiliser is from 5 to 30 L/ha.

20. The method according to claim 19, wherein said application dose is from 10 to 20 L/ha.

21. The method according to any one of claims 17-20, wherein said plant is selected from the group consisting of horticultural plants, ornamental plants, fruit plants, and cereals.

22. A method for obtaining a liquid biofertiliser, comprising:
(a)culturing a strain of *Azospirillum brasilense* deposited in the Spanish Type Culture Collection (CECT) with deposit number CECT 5802 and a strain of *Pantoea dispersa* deposited in the CECT with deposit number CECT 5801 in separate and appropriate first liquid fermentation media for each strain, at a temperature from 25°C to 35°C, under stirring and aeration and
(b) adding in cold the fermentation broths obtained in the previous step to a second fresh liquid medium, previously sterilized.

23. The obtention method according to claim 22, wherein the biofertiliser is an aqueous suspension.

24. The obtention method according to claim 22 or 23, wherein the first liquid fermentation media comprise at least one ingredient selected from the group consisting of tomato paste, collagen hydrolysate, and yeast extract.

25. The obtention method according to any one of claims 22-24, wherein the first liquid fermentation media comprise tomato paste, collagen hydrolysate, and yeast extract.

26. The obtention method according to any one of claims 22-25, wherein the first liquid fermentation media further comprise at least one inorganic salt selected from the group consisting of K₂HPO4, KH₂PO₄, and NH₄Cl.

27. The obtention method according to any one of claims 22-26, wherein the first liquid fermentation media comprise the inorganic salts K₂HPO₄, KH₂PO₄ and NH₄Cl.

28. The obtention method according to any one of claims 22-27, wherein the first liquid fermentation media comprise K₂HPO4, KH₂PO₄, NH₄Cl, tomato paste, collagen hydrolysate, and yeast extract.

29. The obtention method according to claim 28, wherein the first liquid fermentation media comprises K₂HPO₄ 1-6% (w/w), KH₂PO₄ 0.01-0.6% (w/w), NH₄Cl 0.02-4% (w/w), tomato paste 1-6% (w/w), collagen hydrolysate 0.02-4% (w/w), and yeast extract 0.02-4% (w/w).

30. The obtention method according to any one of claims 22-29, wherein the second liquid fresh media comprises at least one inorganic salt, a soluble concentrate of molasses, at least one pH regulating agent, and at least one antifoaming agent.

31. The obtention method according to claim 30, wherein said inorganic salt is selected from the group consisting of (NH₄)₂SO₄, K₂HPO₄, ZnSO₄, MnSO₄, CuSO₄, MgSO₄, and CaCl₂.

32. The obtention method according to any one of claims 22-31, wherein the second fresh liquid medium comprises the inorganic salts (NH₄)₂SO₄, K₂HPO₄, ZnSO₄, MnSO₄, CuSO₄, MgSO₄ and CaCl₂.

33. The obtention method according to any one of claims 30-32, wherein the second fresh liquid medium comprises the pH-regulating agents malic acid and KOH.

34. The obtention method according to any one of claims 30-33, wherein said antifoaming agent is polypropylene glycol.

35. The obtention method according to any one of claims 22-34, wherein the second fresh liquid medium comprises (NH₄)₂SO₄, K₂HPO₄, ZnSO₄, MnSO₄, CuSO₄, MgSO₄, CaCl₂, soluble concentrate of molasses, malic acid, KOH and polypropylene glycol.

36. The obtention method according to claim 35, wherein the second fresh liquid medium comprises (NH₄)₂SO₄ 1-7% (w/w), K₂HPO₄ 0.001-0.2% (w/w), ZnSO₄▪7H₂O 0.001-0.2% (w/w), MnSO₄▪H₂O 0.001-0.2% (w/w), CuSO₄▪5H₂O 0.001-0.02% (w/w), MgSO₄▪7H₂O 0.001-0.02% (w/w), CaCl₂▪2H₂O 0.001-0.02% (w/w), soluble concentrate of molasses 0.1-2% (w/w), malic acid 0.02-0.2% (w/w), KOH 0.01-2% (w/w) and polypropylene glycol 0.001-0.2% (w/w).

37. The obtention method according to claim 36, wherein the second fresh liquid medium comprises (NH₄)₂SO₄ 3-5% (w/w), K₂HPO₄ 0.04-0.06% (w/w), ZnSO₄▪7H₂O 0.02-0.04% (w/w), MnSO₄▪H₂O 0.02-0.04% (w/w), CuSO₄▪5H₂O 0.002-0.004% (w/w), MgSO₄▪7H₂O 0.006-0.008% (w/w), CaCl₂▪2H₂O 0.002-0.004% (w/w), soluble concentrate of molasses 0.5-1.5% (w/w), malic acid 0.07-0.09% (w/w), KOH 0.04-0.2% (w/w) and polypropylene glycol 0.01-0.02% (w/w).

38. The obtention method according to any one of claims 22-37, wherein from 20% to 30% (w/w) of the fermentation broths obtained in step (a) are added to said second fresh liquid medium.

39. The obtention method according to any one of claims 22-38, wherein the cultures of step (a) are performed for a period of time from 8 to 20 hours.

40. The obtention method according to any one of claims 22-39, wherein the stirring rate in step (a) is from 400 to 800 r.p.m.

41. The obtention method according to any one of claims 22-40, wherein the aeration in step (a) is from 0.5 to 2 L/min.

42. A liquid biofertiliser obtainable according to the obtention method of claims 22-41.
